Europäisches Patentamt

(19) European Patent Office

Office européen des brevets

(11) Publication number: **0 212 880**
A1

# EUROPEAN PATENT APPLICATION

(21) Application number: 86305869.9

(22) Date of filing: 30.07.86

(51) Int. Cl.⁴: **C 12 P 21/00,** C 12 N 5/00, A 61 K 39/00

(30) Priority: 01.08.85 IL 75994

(43) Date of publication of application: 04.03.87 Bulletin 87/10

(84) Designated Contracting States: AT BE CH DE FR GB IT LI LU NL SE

(71) Applicant: **State of Israel Represented by the Prime Minister's Office The Israel Institute for Biological Research, P.O. Box 19, Nes Ziona (IL)**

(72) Inventor: **Epstein, Nava, Ben Yehuda St. 1, Petach Tikva (IL)**
Inventor: **Ariel, Naomi, Rothschild Street 30, Nes Ziona (IL)**
Inventor: **Reuveny, Shaul, Bertonov 6 Kfar Ganim, Petach Tikva (IL)**
Inventor: **Altboum, Ze'ev, Bilu Street 11, Petach Tikva (IL)**
Inventor: **Lazar, Arye, Hanasi Harishon 34, Rehovot (IL)**

(74) Representative: **Matthews, Heather Clare et al, Keith W Nash & Co Pearl Assurance House 90-92 Regent Street, Cambridge CB2 1DP (GB)**

(54) Production of carcinoembryonic antigen.

(57) A process for the production of carcionembryonic antigen (CEA), comprises the steps of cultivating a CEA-producing cell line in a monolayer culture; further cultivating the said cell line on particulate microcarriers with continuous agitation of the culture medium, in at least one further cultivation step; harvesting the medium containing the desired CEA secreted by the cells; and purifying the CEA until iodination grade product is obtained.

EP 0 212 880 A1

Title; Production of carcinoembryonic antigen

FIELD OF THE INVENTION

The present invention relates to a process for the production of iodination grade carcinoembryonic antigen (CEA), e.g. from a human colon adenocarcinoma cell line.

BACKGROUND OF THE INVENTION

Carcinoembryonic antigen (CEA) is an onco-fetal glycoprotein, first described by Gold and Freedman [J. Exp. Med. 121: 439 (1965)]. It is normally present in the gastrointestinal tract of the human fetus, but production therein decreases as the fetus matures. CEA may also appear in the serum of adults, and reach high concentrations, particularly in patients with tumors of the gastrointestinal tract, breast, lung, ovary and pancreas. Elevated CEA levels have also been associated with certain non-malignant conditions, inflammatory diseases and chronic pulmonary diseases.

While these findings have precluded the use of CEA assays as an independent test to establish the diagnosis of cancer, such assays are used successfully for serial monitoring of patients with diagnosed cancers. Clinical relevance of the CEA assay has been best documented in the follow-up management of patients with colorectal carcinoma. The assay has also been shown to be of value for serial monitoring of CEA levels in patients with adenocarcinoma arising in other digestive system organs and in the lung, breast and prostate, as well as in patients with epidermoid carcinoma of the lung, esophagus and genito-urinary tract.

The potential importance of such a marker has stimulated the development of various assay techniques and the commercial production of assay kits. The significance of plasma CEA determination is manifested in the following cases:-

(1) Monitoring the patient post-surgically: a persistent rising or high CEA level would suggest incomplete removal of the tumor or the presence of metastatic disease, while a declining CEA level would suggest a successful resection. Elevated CEA levels would generally be associated with a poor prognosis.

(2) Monitoring the response to a regimen of antineoplastic drugs or radiation therapy, when a declining CEA level would suggest a therapeutic response.

(3) Assessing the status of malignant disease locally or at a distant site (metastases) - elevated CEA values have frequently been the only indicator of tumor recurrence, appearing months before other clinical evidence of disease progression.

(4) Radioimmunodetection of metastases-labelled anti CEA-IgG is employed in vivo, for tumor localization and early detection of metastases.

After a CEA baseline is established for a patient, CEA determinations at regular intervals aid in monitoring the course of disease. CEA values are then used in conjunction with information available from the patient's clinical evaluation.

While CEA RIA/ELISA diagnostic kits have been marketed by a number of companies, and such kits are largely consistent in their ability to detect CEA, they nevertheless suffer from a number of drawbacks. Thus, absolute values cannot be compared one with another, due to the heterogeneity and lack of standardization of reagents, of which one is CEA, which is isolated from human hepatic metastases and polyclonal anti-CEA antibodies. Moreover, the supply of such kits is often delayed due to the fact that tissues of human origin (i.e. hepatic metastases) are not always available.

Standardization of the CEA assay reagents is critical for the purpose of clinical studies, and this could be achieved by either (i) employing antibodies of high specificity, purity and reproducible quality, i.e. monoclonal antibodies (which could also be employed in radioimmunodetection and CEA purification); as well as (ii) replacing the heterogeneous metastatic CEA with a highly homogeneous, reproducible and uniform preparation of cell-line CEA, purified from a cell line cultivated in tissue culture, and thus providing long-term consistency of the assay for CEA. As will appear hereinafter, the process of the present invention relates to option (ii).

Cell surface components, secreted into the medium when cells are cultivated in tissue culture, provide a readily available source of antigen of reproducible quality, in essentially unlimited amounts. The prerequisites for the commercial production of antigen in this way are:

(a) large scale, long-term cultivation with constant harvesting of the product;

(b) a purification procedure including the handling of large volumes of spent medium; and

(c) establishing the suitability of the product with reference to its immunological and physico-chemical properties.

Several CEA-producing cell lines have been reported in the literature [see in particular Leibovitz et al, Cancer Res. 36: 4562 (1976)]. These cell lines can be caterogized according to several parameters, including the production of CEA. Production of CEA (as tested by RIA/ELISA kits) was found to depend on the nature of the line as well as on the growth phase, reaching a maximal value at the stationary phase, ranging from 8 to 7500 ug. CEA per $10^6$ cells per 21 days in monolayer culture.

Isolation and characterization of the immunoreactive substance (CEA) has been hampered by the difficulty of long term cultivation of large amounts of cells by conventional methods, necessary for employing cell line CEA as standard antigen in CEA RIA/ELISA kits. Abbott Laboratories was the first and only company to report, in a commercial pamphlet (1981), that CEA employed as a standard in clinical assays had been isolated from a cell line, but this pamphlet provided no details as to the cultivation and purification procedure.

Adaptation of one of the recently developed modes of cultivation of anchorage dependant cells (hollow fibers,

microcarrier culture etc.,[Quarales et al, In Vitro, 16: 113 (1980)], by providing a large cell growth surface area in a small volume, would overcome the obvious complications involved in large scale cell cultivation in monolayer culture. In the microcarrier culturing system [Reuveny et al, Dev. Biol. Standards, 50: 115-23 (1982)], cells are propagated on small solid particles suspended in culture medium, with slow agitation, in (e.g.) spinner flasks. This system is successfully used for the cultivation of various cell lines. A CEA-producing cell line (as determined by CEA RIA) was reported by Page et al (Pharmacia booklet on microcarrier culture) to grow on Cytodex-1 microcarriers (Pharmacia) and in Petri dishes. The culture was not scaled up, and the antigen has not been purified. The microcarrier system was successfully applied by the Applicants to the large scale, long term cultivation of a sub-clone, HuCCL-14A, isolated following dilution and cultivation in soft agar, of an established CEA-producing cell line [Yaniv et al, Exp. Cell Biol., 46: 220-230 (1978)]. The Applicants have developed a procedure for the purification of the antigen and established its identity to metastatic CEA, in terms of its immunoreactivity and physico-chemical properties.

Adaptation of the growth of the cell line onto microcarriers required screening for the optimal microcarrier which allows for both maximal growth and maximal secretion of CEA. Screening was carried out in stationary cultures, and consideration was given to commercially available carriers, as well as to cylindrically-shaped microcarriers developed by the

Applicants. From the results, it was evident that although the amount of CEA produced was comparable, nevertheless beaded microcarriers cannot support long term cell growth, since cells grow as aggregates outside the microcarriers. Cylindrically-shaped rigid cellulose microcarriers were found to support cell growth in aggregates entrapping the microcarriers. When tested in submerged cultures, with agitation, e.g. in spinner flasks, it was found in accordance with the present invention, that certain cellulose microcarriers were most suitable, forming aggregates which resulted in high cell densities (and thus high levels of CEA) and a homogeneous suspended culture. CEA levels increased with cell growth, reaching a maximum (within the range of 0.2 to 0.8 mg. per liter of culture) after up to 15 days. In contrast with monolayer culture, it was found that CEA levels did not drop, but remained constant over a period of 3 months, at least, with 50% cell viability.

The advantages of the microcarrier/agitated medium culturing system are that:-

(1)    The CEA can be harvested from submerged cultures, running continuously over an extended period of time.

(2)    Various environmental factors such as aeration and CEA-secretion modifiers can be monitored and controlled with respect to their effect on cell growth and CEA secretion.

(3)    The system can be scaled up.

In purifying CEA from spent medium, the aim should be a procedure which is easy to perform with large volumes of low specific activity. Most of the commonly used methods for the purification of CEA from human hepatic metastases are based on a method described by Krupey et al [Immunochemistry, 9: 613 (1972)], consisting of perchloric acid (PCA) extraction, two gel filtration steps and preparative electrophoresis. Several groups have subsequently modified the procedure, employing ion-exchange columns and affinity chromatography, based on either polyclonal or monoclonal antibodies. These modifications allow rapid and selective purification of CEA from samples of large volume and low specific activity (i.e. from spent media).

In accordance with an embodiment of the present invention, Applicants have developed a procedure for the purification of cell line CEA based on the above principles, but utilizing preferably PCA and ammonium sulfate extractions, and affinity chromatography on monoclonal anti-CEA antibodies. The purification factor is of the order of 16,700, yielding iodination grade CEA at 35-45% yield. (see Table A, infra). The purified antigen is uniform, readily available and of reproducible quality. It is therefore ideally suited for use as a standard in a clinical kit, provided that its properties are comparable to the established standard metastatic CEA.

Due to the lack of any known function of CEA, and its heterogeneous physicochemical properties, new CEA preparations are evaluated by a combination of recommended physicochemical and immunological criteria, namely:-

7

(1)   A single diffuse component with an apparent MW of 180,000-200,000 on SDS-PAGE, similar to metastatic CEA.

(2)   The purified antigen should give a reaction of identity in double immuno-diffusion analysis with a known CEA standard, using previously characterized antisera.

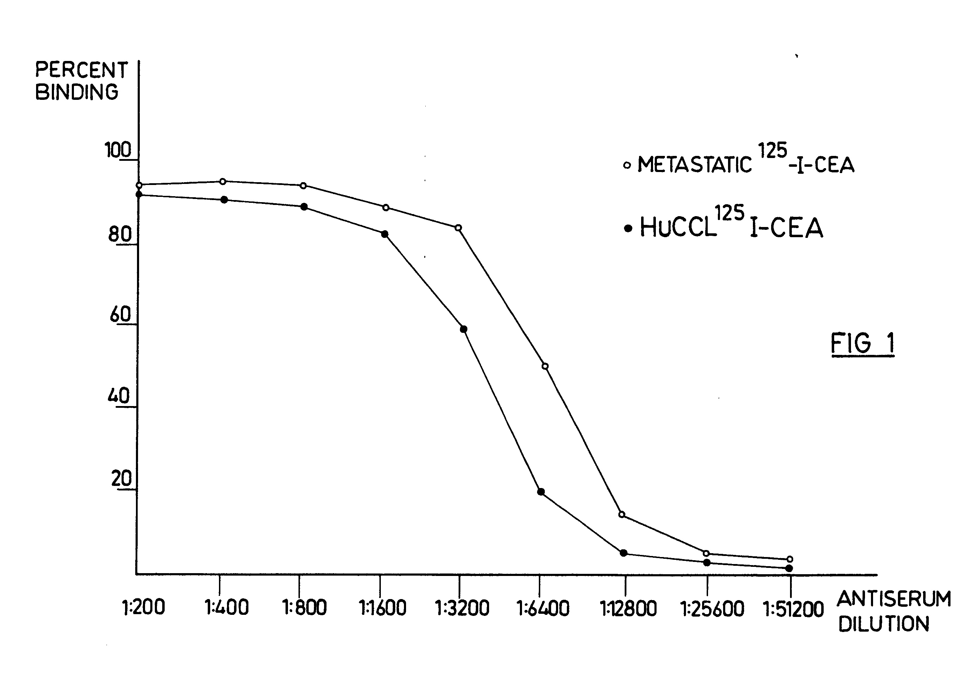

(3)   The 125I-labelled CEA should give a typical binding curve with an established antiserum (Fig. 1).

(4)   The antigen should give an activity in CEA RIA/ELISA for CEA which is similar to other CEA preparations.

(5)   The antigen should be a glycoprotein, composed of 40-60% carbohydrate and a single polypeptide chain, with a typical and reasonably constant carbohydrate composition and linkage analysis (Tables 1 and 2).

The cell line CEA produced in accordance with an embodiment of the invention was found to fulfill the foregoing criteria, and accordingly the product is especially suitable for the purposes described above.

It should be emphasized that Applicants' purpose was to produce a CEA preparation, which according to the recommended criteria is indistinguishable from metastatic CEA, and is thus capable of replacing metastatic CEA in commercial kits. (Minor structural differences will always be observed between metastatic and cell line CEA, as well as between different batches of metastatic CEA). The advantages of cell line CEA over metastatic CEA are homogeneity, reproducibility and ready availability.

The relevant problems (overcome by the Applicants) were the adaptation of cultivation of the cell line for long term,

8

large scale production of crude CEA, purifying the product by a convenient, efficient and reproducible procedure, and establishing the identity of cell line CEA with metastatic CEA.

## SUMMARY OF THE INVENTION

The present invention accordingly provides a process for the production of carcinoembryonic antigen (CEA), comprising the steps of

cultivating a CEA-producing cell line in a monolayer culture;

further cultivating the said cell line on particulate microcarriers with continuous agitation of the culture medium, in at least one further cultivation step;

harvesting the medium containing the desired CEA secreted by the cells; and

purifying the CEA until iodination grade product is obtained.

The process may be effected batchwise or continuously. Preferably, at least the further cultivating and isolating steps are effected as part of a continuous process. It is particularly preferred that the harvesting step is effected on only part of the spent medium, and the removed medium is replaced by a similar amount of fresh medium, thus maintaining continuous growth and production of the desired product. This harvesting step is most preferably repeated periodically at intervals of several days.

The microcarrier is preferably a cellulose-derived

9

microcarrier, and the microcarrier is also preferably rigid and either cylindrically or spherically shaped.

As regards the monolayer cultivation step, this may be effected for a period of 3-5 days or until confluency is reached. Any suitable culture medium may be used, but for this purpose it is preferred to use RPMI No. 1640. The medium may comprise additionally an antibiotic, e.g. one which is selected from the group consisting of neomycin, penicillin, streptomycin or gentamycin. Moreover, the culture medium may also comprise additionally fetal bovine serum (FBS), which is preferably present in a proportion of substantially 10% of the RPMI No. 1640. The pH of the culture medium is preferably adjusted to a pH value of about 7.2, and a suitable operational temperature for this step is 37 (+/-) 5 °C, preferably substantially 37 °C.

The monolayer cultivation step is suitably conducted in an environment having a carbon dioxide content greater than that normally present in the atmosphere; for example, this environment may comprise about 5-10% $CO_2$ in compressed air.

Following monolayer cultivation, the desired cells may be removed from the surface culturing vessel with a trypsinization solution, e.g. with a Trypsin-EDTA solution.

As regards the at least one further cultivation step, this may be effected (for example) for a period of 3-10 days or until confluency is reached. The culture medium for this at least one further cultivation step may be any suitable medium for this purpose, but preferably comprises RPMI No. 1640, and more preferably also fetal bovine serum (FBS), which at least initially may be present in a proportion of e.g., substantially 10% of the RPMI No. 1640. A suitable pH for the medium is about 7.2. A suitable operational temperature is 37 (+/-) 5 $^{\circ}$C, preferably substantially 37 $^{\circ}$C. The culture medium may be aerated with a mixture of carbon dioxide and air.

In one embodiment of the process of the invention, the at least one further cultivation step comprises at least two such successive steps of which the volume of operation in the second of said steps is approximately a whole number multiple (e.g. about 5-10 times), of the volume of operation in the first of said steps.

The isolation step may comprise extraction with perchloric acid and rejection of the insoluble matter. This extraction may be followed by concentration and desalting, and the latter operation may be followed by treatment with ammonium sulfate, and rejection of the non-CEA proteins thus precipitated. The treatment with ammonium sulfate may be followed by further concentration and desalting. In one embodiment of the process of the invention, the purification step is immediately preceded by the additional steps of ultrafiltration and centrifugation.

The purification step itself is preferably effected by affinity chromatography, more preferably by affinity chromatography on a Sepharose monoclonal-Anti-CEA-Ab column. A

11

suitable temperature for this operation is about 4°C.

There is also provided in accordance with the present invention carcinoembryonic antigen with an immunoreactivity similar to established CEA preparations, having a molecular weight substantially in the range of about 160,000-200,000 and possessing the following % by weight analysis, namely: protein, 45.2 (+/- 2.3); N-acetylglucoseamine, 19.1 (+/- 0.95); mannose, 6.6 (+/-0.33); fucose, 7.6 (+/- 0.38); galactose 9.3 (+/- 0.47); and sialic acid 12.3 (+/- 0.62). The invention also extends to carcinoembryonic antigen when prepared in accordance with the process described and claimed herein.

## DETAILED DESCRIPTION OF THE INVENTION

I.  The Cell Line.

The CEA is produced in accordance with the invention by cells from an established cell line derived from a colon adenocarcinoma patient. Suitable cell lines are, by way of example, those which have been deposited with the American Type Culture Collection under the accession numbers ATCC CCL 229 LoVo, ATCC CCL 233 SW1116, ATCC CCL 235 SW837 and ATCC CCL 238 SW1417, as well as those described by Yaniv et al in Exp. Cell Biol., 46:220-230 (1978). The preferred cell line in the exemplified embodiment of the invention is designated HuCCL-14A, viable samples of which are available to the public from the Israel Institute For Biological Research, P.O. Box 19, Nes Ziona, Israel. These cells are anchorage-dependant cells and grow only when adhereing to solid surfaces.

## II.  Preparation of Cell Stock.

Cells are grown in any available monolayer culture vessel (e.g., T-flasks, Roux bottles or other monolayer culturing vessels). For each surface area of $100cm.^2$, inoculation of at least $5 \times 10^6$ cells is required. The medium is composed of RPMI No.1640, 1040 parts; $NaHCO_3$, 200 parts; neomycin

penicillin, streptomycin, gentamycin or other antibiotic, 8 parts. It is brought to pH 7.2 with NaOH, and supplemented with 10% fetal bovine serum (FBS). Cultures are incubated at $37^o$C in a $CO_2$ incubator (5-10% $CO_2$ in compressed air).

After 3-5 days of incubation, when confluency of the cells is observed, cells are removed from the surface culturing vessel with any trypsinization solution such as Trypsin-EDTA solution [g./l. of NaCl 8.0; KCl 0.4; glucose 1.0; $NaHCO_3$ 0.58; trypsin (1: 300) 0.5; EDTA 0.2]. After decantation of the supernatant, 0.2 ml. trypsinization solution is added to each 100 ml. of monolayer culture medium.

The cells are collected in 50 ml. conical centrifuge tubes, with 10 ml. RPMI No. 1640 medium and 10% added FBS. The tubes are centrifuged at 500 g for 10 minutes and the supernatant is decanted. The cell pellet is retained for further processing. To each pellet is added 10 ml. RPMI No. 1640 medium and 20% added FBS. The cell concentration is diluted to 2-5 x $10^6$ cells/ml. To the cell suspension, dimethylsulfoxide is added to a final concentration of 10.7% v/v. Aliquots of 3-4 ml. cell suspension are frozen at $-70^o$C or in liquid nitrogen until required for use. The freezing is first effected slowly, at the rate of about $1^o$C per minute until the temperature reaches -5 to $-10^o$C, and then rapidly.

III. Thawing of Cell Stock.

For the cultivation of cells from stock, a stock tube is rapidly thawed in a $37^o$C water bath. To the cell suspension, 5-7 ml. of pre-warmed (37°C) RPMI No. 1640 medium supplemented with 10% FBS are added. The cell suspension is centrifuged at 500 g for 10 minutes, the supernatant is decanted, and each pellet is resuspended in 10-15 ml. RPMI No. 1640 medium supplemented with 10% FBS. The cell suspension is inoculated in a T-flask of surface area 75 cm$.^2$. The culture volume is adjusted to 25 ml. with the same medium. The culture medium is then incubated at 37°C under $CO_2$ for three days or until confluency is reached. The supernatant is replaced daily with fresh medium. The culture is used for the preparation of a CEA production line in a microcarrier culturing system.

### IV.  <u>CEA Production Line.</u>

In the CEA production line the cells are grown on microcarriers which are suspended in a growth medium in controlled, aerated and/or agitated culture in spinner flasks.

### (1)  <u>Microcarriers for CEA Production.</u>

Several types of microcarriers were tested with a view to selecting the optimal carrier in terms of growth of the CEA-producing cells and secretion of CEA. Microcarriers tested included Cytodex-1 and Cytodex-3 of Pharmacia, SuperBeads of Flow Laboratories, Biosilon of Nunc, and DE-52 and DE-53 of Whatman. DE-53 was found to be the best for both cell growth and CEA secretion. DE-53 is a microgranular, rigid and cylindrically shaped DEAE-cellulose anion exchanger having an exchange capacity of 2 m.-eq. per g. of dry material. The DE-53 was preswollen before use.

(2)  Preswelling of Microcarrier.  IS         0212880

To 15 g. DE-53 were added 100 ml. of Phosphate Buffer Solution (PBS) at pH 7.2, which contained (g./1): NaCl 8.0; KCl 0.2; $Na_2HPO_4$ 1.15; $KH_2PO_4$ 0.2. The DE-53 was resuspended and after settling, the process was repeated twice more. The pH of the suspension was adjusted each time to 7.2. The volume of the suspension was brought to 100 ml. with PBS. This suspension was sterilized in presiliconized bottles, for 20 minutes with steam at $121^{\circ}C$. The sterilized DE-53 suspension was kept at $4^{\circ}C$ until use.

(3)  Building-up of CEA Production Line.

This was effected stepwise, as follows:

Step 1: propagation of cells in a monolayer culture.

This is effected in any available monolayer culture vessels, e.g., T-flasks (25, 75 or 150 $cm.^2$ surface area), Roux bottles (200 $cm.^2$ surface area), or other monolayer vessels. The initial concentration in all such vessels was about 1 x $10^5$ cells/$cm.^2$, and the growth medium was RPMI No. 1640 medium supplemented with 10% FBS, added in an amount of 25 ml. per 100 $cm.^2$ surface area. The source of cells may be any monolayer or microcarrier culture of cells thawed from stock. The culture medium was incubated at $37^{\circ}C$ under $CO_2$ for 3-5 days, or until confluency was achieved.

Removal of cells from the solid surface is effected by trypsinization with Trypsin-EDTA solution in an amount of 0.2 ml./ml. of culture medium. 1 ml./ml.(of trypsinized cell suspension) of RPMI No. 1640 medium supplemented with 10% FBS was

15

added, and the suspension centrifuged at 500 g for 10 minutes. The supernatant was discarded, and the residual cell pellet was retained for further processing as the seed of cells, for cell cultivation for the purpose of CEA production for the following build-up steps.

Step 2: propagation of cells in one liter microcarrier agitated culturing vessels.

Microcarrier suspension (7 ml.) is added to 100 ml. final culture volume. Trypsinized cells from 5-10 flasks (each of 150-200 cm.$^2$ surface area) are used as seed for one liter microcarrier culture. Initial growth medium is RPMI No. 1640 medium supplemented with 10% FBS. The microcarrier culture medium was incubated under continuous magnetic agitation at a speed of 30-50 r.p.m. (It has been found that under these conditions the cells grow in multilayers in the microcarrier and form aggregates of microcarriers with cells). The cells were counted daily using the Sanford method for counting the nuclei [J. Natl. Inst. 11: 773 (1951)]. For this purpose, cells are removed by treating the microcarrier/cell aggregates with a solution containing 1.0 g./l. crystal violet and 42.0 g./l. citric acid, prewarmed to 37 °C, and incubating for one hour at the same temperature. The suspension was mixed efficiently to liberate the nuclei from the cells; the number of counted nuclei represented the number of cells in the microcarrier culture medium. For the assay of CEA, the daily agitation of the culture was stopped, the microcarrier allowed to settle, and a sample of the supernate removed.

Step 3: After 3-10 days, confluency of the microcarrier with the cells was achieved and the CEA production culture was scaled up to 6-8 liters microcarrier culture in spinner flasks. Two to four microcarrier cultures (each of 1 liter) were pooled to provide one 6-8 liters microcarrier culture, the desired volume being achieved with RPMI No. 1640 medium supplemented with 10% FBS. The required quantities of fresh preswollen microcarrier were also added. The cultures were bottom aerated with a mixture of 5% $CO_2$ and compressed air at a rate of 0.5-2 liters per minute. The cultures were magnetically stirred at 30-60 r.p.m. Cell counts were effected daily, and after confluency of the microcarrier with cells was achieved, the supernate was harvested for CEA assay and purification.

In order to harvest the supernate, the microcarrier cultures were incubated without stirring for 30-60 minutes, the cell/microcarrier aggregates sedimented, and 30 to 60% of the supernate (free of aggregates) harvested. The supernate containing crude CEA was stored at $-20^{\circ}C$ until required for further processing (e.g. concentration and purification). Fresh growth medium corresponding in volume to the harvested supernate was added to the culture vessel, and growth continued. Harvesting of the 6 liter vessels is effected every two to three days.

As the culture medium becomes older, and confluency is achieved generally, the FBS concentration in the added growth medium is lowered towards 2%. Typical cell counts in the microcarrier cell cultures are (whatever the scale of production): initial cell concentration: $1\text{-}2 \times 10^5$ cells /ml.; at confluency: $3\text{-}4 \times 10^6$ cells/ml.

(4) <u>Purification of CEA.</u>

This was effected in accordance with the following steps.

<u>Step 1:</u> Crude CEA in the form of culture supernate, which had been frozen at $-20^{\circ}$ C, was thawed at 4, 22 or $37^{\circ}$ C, and pooled to batches of 30-100 liters. To the latter contained in a vessel, perchloric acid was added to a concentration of 1 molar, the solution mixed well for 20 minutes; non-specific proteins were precipitated, while the CEA remained in the solution.

<u>Step 2:</u> The product from step 1 was centrifuged continuously at 5000 g, the precipitate was discarded, and the supernate was recovered; the pH of the latter was adjusted to 7.0 with ammonium hydroxide.

<u>Step 3:</u> The CEA was concentrated from the neutralized supernate by ultrafiltration at $4^{\circ}$ C, either batchwise or continuously, using a 100,000 dalton cut-off membrane. This step was terminated when the crude CEA was concentrated in a volume of 2-4 liters.

<u>Step 4:</u> Desalting of the crude concentrated CEA was effected in a closed system using demineralized water. Desalting was terminated when the conductivity of the solution reached 10 uMho. The concentrated desalted CEA solution occupied 2-4 liters.

Step 5: Precipitation of unwanted non-CEA proteins was done by continuous addition of ammonium sulfate to a total of 315 g./l. CEA solution. Stirring was effected magnetically until all the salt dissolved. The solution was acidified to pH 2 by addition of concentrated sulfuric acid. Magnetic stirring was continued at 4$^{\circ}$C for 14-20 hours, and the mixture was then centrifuged at 5000 g for 30 minutes at 4$^{\circ}$C. The precipitate containing unwanted proteins was rejected and the supernate containing the desired CEA was retained.

Step 6: The CEA was concentrated from the supernate by batchwise ultrafiltration at 4$^{\circ}$C, for 14-18 hours, using a 100,000 dalton cut-off membrane.

Step 7: Desalting of the concentrated CEA was effected with PBS at 4$^{\circ}$C. Desalting was terminated when the conductivity of the solution reached that of the PBS; the duration of this step was 14-18 hours. The volume of the CEA solution was about 1 liter after steps 5-7; it was stored at -20$^{\circ}$C, pending further processing.

Step 8: Purification of the CEA prior to treatment on a monoclonal anti-CEA affinity chromatography column.

Frozen CEA is thawed to 4$^{\circ}$C, residual precipitates are removed by centrifugation at 4$^{\circ}$C at 5000 g for 30 minutes, the product is submitted to batchwise ultrafiltration at 4$^{\circ}$C, for 14-18 hours, using a 100,000 dalton cut-off membrane, and is finally submitted to centrifugation for 30 minutes at 10,000 g to remove residual precipitates.

Step 9: Production of monoclonal antibodies.

Hybridoma cell-secreting monoclonal anti-CEA antibodies were produced by the fusion technique according to Kohler and Milstein (Nature, 256: 495-497 (1975)). Two monoclonal antibodies were chosen for the affinity purification process. Monoclonal antibodies from other cell lines have previously been used in a similar manner for CEA purification, see e.g. Rogers et al, Br. J. Cancer, 44:371 (1981) and Buchegger et al, Proteins and Related Subjects (Peeters (ed.), Brussels), 28:511 (1980).

Step 10: Hybridoma Growth in Mice; Monoclonal Antibody Isolation.

Balb/c mice were injected intraperitoneally with 0.5 ml. pristane (2,6,10,14-tetramethylpentadecane), an immunsouppressive substance, at least 7 days prior to an anticipated injection of hybridoma cells. Hybridomas ($10^6$ - $10^7$) cells per mouse) were then injected into Balb/c mice intraperitioneally, where an antibody-rich ascites fluid develops. Seven to fourteen days after injecting the cells, the mice exhibit substantial abdominal swelling, and the ascites fluid is removed into test tubes using an 18.6 x 1.5 sterile needle. Subsequently, the ascites are allowed to clot, and are clarified from the fibrin by filtration over glass wool.

Step 11: Affinity Separation on Protein-A-Sepharose.

Protein-A-Sepharose CL 4B (Pharmacia, Uppsala) (15 g.) is subjected to swelling in 0.05 M Tris/0.15 M NaCl pH 8.0 containing 0.02% $NaN_3$ (= buffer "a"). The resin is packed in a small column: a 10 ml. syringe, with glass wool at the lower end. Acites fluid is dialyzed against buffer "a" for 1-2 hours at 4°C. The dialyzate is centrifuged at 10,000 g to precipitate denatured protein. The supernate is applied to the column (of capacity about 50 mg. Ab), unbound protein is washed off with buffer "a" (a UV monitor may be used to follow the elution). Fractions of bound protein are eluted using the following gradient. The initial gradient buffer ("b") consists of 0.1 M Tris/0.15 M NaCl containing 0.02% $NaN_3$ and titrated to pH 2 with 1 M HOAc. The limiting gradient buffer ("c") consists of 0.1 M HOAc/NaOAc + 0.15 M NaCl at pH 3, and containing 0.02% $NaN_3$. 100 ml. each of buffers "a" and "b" are utilized. 2 ml. fractions are eluted into test tubes containing 0.4 ml. 1 M Tris at pH 8 for neutralization. The monoclonal anti-CEA Ab, which is of $IgG_{2b}$ subclass is located in fraction No. 3. The column is regenerated by washing with buffer "c", and then re-equilibrated with buffer "a".

Step 12:  Preparation of Affinity Column Sepharose Monoclonal-Anti-CEA-Ab.

The purified monoclonal anti-CEA Ab is concentrated and dialyzed in an ultrafiltration cell (Amicon) against coupling buffer (0.1 M $NaHCO_3$ / 0.5 M NaCl at pH 8.3), using an Amicon UM-30 membrane. CNBr-activated Sepharose 4B (Pharmacia) (3 g.) is swollen by treatment for 15 minutes in 200 ml. 1 mM HCl, centrifuged for 7 minutes at 1000 g, washed with coupling buffer and brought to a final volume of 20 ml., including 10.5 ml. swollen resin. To this is added 50-100 mg. purified monoclonal anti-CEA Ab in coupling buffer, and the volume is made up to 45 ml. therewith; the test tubes are mixed gently using a head over end rotator for 2 hours at room temperature.

The resin is centrifuged for 7 minutes at 1000 g. The OD was read at 280 nm to evaluate protein contents (over 90% of the monoclonal anti-CEA Ab should remain bound to the resin). The resin precipitate is suspended in 40 ml. glycine buffer at pH 8, for 2 hours at room temperature, in order to block access of reactive groups which did not bind the monoclonal anti-CEA Ab. In order to remove monoclonal anti-CEA Ab which is non-covalently attached to the resin, the latter is washed with 50 ml. 0.1 M acetate buffer containing 0.5 M NaCl, at pH 4, followed by washing with 50 ml. of coupling buffer; this process is repeated five times.

Step 13: Affinity Purification of CEA.

A solution containing 10 mg. CEA in PBS is concentrated to 100 ml. This solution is incubated with Sepharose monoclonal-Anti-CEA-Ab, while rotating end over head for 24-48 hours, at 4°C, the resin is placed in a column (1 x 15 cm.), and washed (PBS + 0.5 M NaCl + 0.02% $NaN_3$), using a peristaltic pump, until no further protein is eluted. The column is then eluted with 0.025 M glycine-HCl buffer containing 0.5 M NaCl at pH 2.5; fractions containing CEA (as monitored by ELISA, VIII), 1.6 ml. each, are collected into test tubes containing 0.4 ml. 1 M glycine buffer at pH 8.

(5) Analysis of Product CEA.

A. Iodination of CEA.

CEA was labelled by a modification of the method of Greenwood et al [Biochem. J. 89: 114 (1973)]. The following

22

were successively added to a small (1 x 3 cm.) stoppered vial equipped with a tiny glass coated metal rod:- 25 ul. of 0.25 M sodium phosphate buffer at pH 7.4; 10-20 ul. of CEA solution (containing 5 ug CEA) in 0.4% $NaHCO_3$; 10 ul. $^{125}I$ (1 mCi) carrier free (Amersham); and 15 ul. of freshly dissolved 0.01% chloramine T in water. After 6 minutes stirring at room temperature, the reaction was terminated by 10 ul. of 0.1% sodium bisulfite solution. After 1 minute, 50 ul. of an aqueous solution of KI (16.6 mg./ml.) plus 130 ul. of 0.25 M sodium phosphate buffer at pH 7.7 were added and the iodinated CEA was separated from the free iodide by gel filtration on a Sephadex G-25 (fine) column, packed in a 10 ml. disposable pipette, and equilibrated with 0.25 M sodium phosphate buffer at pH 7.4 with 2% serum albumin. 0.5 ml. samples of the eluate were collected into tubes containing 0.1 ml. of 2% aqueous BSA solution. The tubes containing the radioactive protein were pooled, diluted with 0.2% BSA in the reaction buffer to approximately $10^7$ cpm/ml. and stored frozen in an aliquot of 0.5 ml. The specific activity of the labelled CEA ranged between 30 and 60 Ci/ug. The binding activity remained stable up to 6 weeks.

B. Characterization of Cell Line CEA.

The product CEA is characterized according to the criteria mentioned in "Background of the Invention", above. Amino acid composition of CEA is set out in Table 1, and overall composition of CEA in Table 2 (infra).

(i) Amino acid analysis was performed according to the method of Stein and Moore, employing a model D-500 Pronex analyzer. Samples were hydrolyzed in evacuated tubes with 6N HCl for 21 hours at 110°C, with added norleucine as an internal standard.

(ii)     Total carbohydrate content was determined by the phenol/sulfuric acid method.

Table 1:  Amino acid composition of CEA (mole %).

| amino acid | CEA (metastatic) | CEA (cell line) |
|------------|------------------|-----------------|
| ASP | 13.5 | 13.1 |
| THR | 8.59 | 8.26 |
| SER | 10.26 | 10.72 |
| GLU | 10.26 | 10.53 |
| PRO | 7.61 | 7.37 |
| GLY | 5.89 | 6.92 |
| ALA | 6.21 | 6.38 |
| 0.5 Cyst. | 1.90 | 1.35 |
| VAL | 6.77 | 6.27 |
| MET | 1.2 | 0.9 |
| ILE | 3.2 | 3.2 |
| LEU | 7.4 | 7.4 |
| TYR | 3.50 | 4.1 |
| PHE | 2.21 | 2.54 |
| HIS | 1.77 | 1.71 |
| LYS | 6.6 | 4.1 |
| ARG | 4.3 | 3.79 |

[Note:    0.5 Cyst. and methionine were determined as cysteic acid and as methionine sulfone after performic acid oxidation.]

Table 2:  Composition of CEA (weight %).          0212880

|                      | CEA (metastatic) | CEA (cell line) |
|----------------------|------------------|-----------------|
| Protein              | 47.7             | 45.2            |
| (Carbohydrate:-)     |                  |                 |
| N-Acetylglucose amine | 26.6            | 19.1            |
| Mannose              | 5.9              | 6.6             |
| Fucose               | 7.5              | 7.6             |
| Galactose            | 10.4             | 9.3             |
| Sialic acid          | 2.0              | 12.3            |
| Molecular weight     | 200,000          | 180,000         |

(iii)     Amino-sugars were measured by aminoacid analysis, after hydrolysis with 4 N HCl for 3 hours at 160$^{\circ}$C according to Fauconnet et al [Anal. Biochem., 91: 403 (1978)].

(iv)     Neutral sugar analysis was performed following sample hydrolysis with 2 N trifluoroacetic acid for 2 hours at 100$^{\circ}$C. Sugars were determined by anion exchange chromatography in borate buffer under conditions similar to those described by Lee [Methods. Enzymol., 28: 63 (1972)].

(v)     SDS PAGE was carried out with apparatus and chemicals supplied by BioRad.  In slab gels 0.75 mm. thick, with a 5-20% linear polyacrylamide gradient, in the buffer system of Laemli [Nature, 727: 690 (1970)]. Gels were fixed and stained with 0.2% (w/v) Coomassie Blue G-250 in 40% methanol (v/v), 10% HOAc (v/v), and destained by diffusion in 7% methanol, 7% HOAc and 1% glycerol (v/v).  Molecular weight markers were from Pharmacia. $^{125}$I-labelled CEA was detected by autoradiography of dried gels using Kodak film.

(vi)     Binding Curve. (Fig. 1)

*26*

The binding of a fixed amount of $^{125}$I-CEA is measured at serial antiserum dilutions, to estimate the titer of the antiserum and the extent of binding, with respect to the particular preparation of labelled antigens, and to evaluate its immunoreactivity (Cell Line CEA v. metastatic CEA).

(a) Anti-CEA IgG of established specificity and titer (horse) is diluted in 0.05 M boric acid-borax buffer, pH 8.5, containing 0.5% (v/v) of normal serum of the same species. Each assay tube contains 500 ul. of diluted antiserum (or only normal serum, in the case of the blank).

(b) About 20,000 cpm $^{125}$I-CEA in 100 ul. of diluent is added. After 2 hours of slow shaking at 37°C, 150 ul. of undiluted anti IgG antiserum (determined to be in excess by pre-titration) is added. After 1 hour at 37°C and 1 hour on an ice-bath (or overnight at 4°C), precipitates are collected by centrifugation at 8000 g (Sorvall H54 rotor) for 25 minutes at 4°C. The precipitates are counted.

(c) The antibody specific binding is calculated for each serial dilution according to the equation:

$$\% \text{ Bound} = [(B - N)/(T - N)] \times 100,$$

where B = cpm of tube; N = blank; and T = total counts.

(d) The antigen/antibody complex may also be precipitated by fixed S. aureus Cowan I.

(e) Ordinarily, 90% or more radioactivity is precipitated, and non-specific counts represent 4-8% of the total. Labelled preparations are usually discarded after 3-4 weeks. Similarity of the two antigen preparations is judged by the extent of maximal binding as well as by the titer obtained.

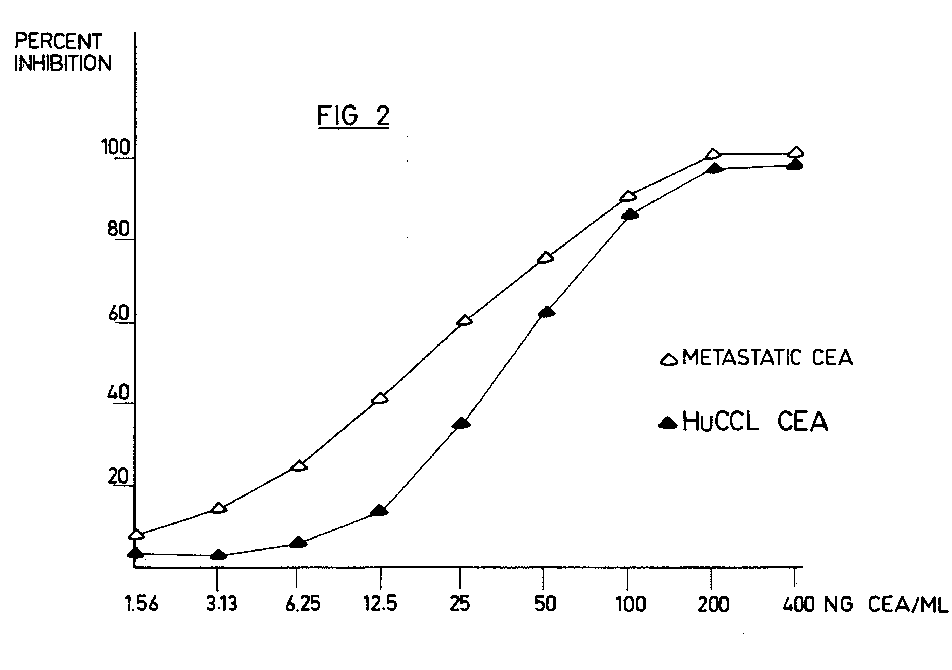

(vii)       Inhibition Curves. (Fig. 2)

(a)   The inhibition assay is carried out at an antiserum dilution such that 50% of the maximal binding occurs when an inhibitor is present.

(b)   Antigens are compared as to maximal inhibition, shape of curve and concentration exhibiting 50% inhibition.

(c)   Each assay tube contains 500 ul. of diluted antiserum and 50 ul. of boric acid-borax buffer diluent (c.f. binding curve) containing 1-200 ng./ml. of unlabelled CEA. The blank tubes contain only diluent and represent the non-specific precipitate. Tubes containing antiserum but no unlabelled CEA represent the maximum precipitation in the absence of inhibitor.

(d)   Standard metastatic CEA is prepared at a concentration of 1.56, 3.12, 6.25, 12.5, 25, 50, 100 and 200 ng./ml. in boric acid/borax buffer, containing normal horse serum (c.f. binding curve) plus 0.01% thiomersal, and these preparations are kept at $4^{\circ}C$ for one month.

(e)   For samples containing unknown amounts of CEA to be assayed, 50 ul. of the appropriate dilution is added instead of CEA standard.

(f)   All tubes are incubated for 2 hours at $37^{\circ}C$, followed by addition of about 20,000 cpm of $^{125}$I-labelled CEA in 100 ul. of boric acid/borax buffer (containing normal horse serum ).

(g)  Subsequent incubations, the addition of second antibody and  the collection of precipitates are carried out as  described above for the binding curve.

(h)  The  inhibition  for  each  standard  or  unknown  is calculated according to the equation:

$$\% \text{ inhibition} = [1- (B - N)/(B_o - N)] \times 100,$$

where Bo = counts bound in the absence of unlabelled CEA.

(viii)  ELISA for CEA.

CEA  concentration  is determined by  the  "double sandwich" method.  The assay comprises the following steps.

(a)  PVC  96-well microtiter plates (Dynatech)  are  coated with polyclonal antiCEA IgG [10 ug.  per ml. in 50 mM Tris-HCl at pH 7.6, containing 0.9% NaCl (TS);  150 ul. per well] for 3 hours, in a humidified chamber.  The excess antiserum is  removed,  the wells  are rinsed 3 x in TS buffer and blocked by treatment  with 150  ul.  per well of TSA (TS buffer containing 1  mg.  BSA/ml.), overnight at $4\,^{\circ}C$.

(b)  All CEA-containing samples are freshly diluted to  1-30 ng.  CEA/ ml.,  in TSA containing 0.05% Tween-20 (TSAT).  Samples containing  high amounts of proteins (i.e.  serum) are  extracted with  0.2 M sodium acetate at pH 5.0;  0.5 ml.  is added to 1 ml. buffer),  for 10 minutes at $70\,^{\circ}C$,  followed by centrifugation  of the  sample  at 12,000 g,  and removal of the supernatant  to  be assayed.

(c) Standard CEA solutions are prepared from pure CEA, and calibrated against a commercial assay (Abbott CEA-EIA). All the dilutions are done in TSAT. Standards containing 0, 2.5, 5, 10, 17.5 and 20 ng./ml. are stable at $4^{\circ}C$, for at least two months, provided that 0.01% thiomersal is added. The stock solution contains 100 ug. CEA/ ml., in buffer, and is kept frozen ($-20^{\circ}C$, in aliquots). The diluted standards should not be frozen.

(d) The assay is performed in duplicates. 100 ul. of CEA (standard or sample) is added to each well. After 90 minutes at $37^{\circ}C$, the antigen is removed and the wells are washed 3 x with TST.

(e) 100 ul. of horse anti-CEA IgG (approximately 10 ug./ml. in TSAT) is added to each well. Following incubation for 90 minutes at $37^{\circ}C$, the wells are rinsed 3 x with TSAT.

(f) 100 ul.of rabbit anti-horse conjugated to HRP (Miles) is added (diluted 1: 2000-5000 in TSAT), followed by incubation for 40 minutes at $37^{\circ}C$, and rinsing 3 x with TST.

(g) 100 ul. of substrate (ABTS + $H_2O_2$) is added to each well (except the blank), and incubated for 20 minutes at room temperature. [ABTS is 2,2'-azinodi(3-ethylbenzenethioline-sulfonic acid), freshly prepared by dissolving 1 mg. in 5 ml. citrate buffer containing 50 ul. $H_2O_2$ (0.3%, freshly prepared); Citrate buffer is 0.1 M citric acid titrated to pH 4 with 0.2 M $Na_2HPO_4$.]

(h) The content of each well is removed to a new microtiter plate and the absorbance is monitored at 405 nm, in an ELISA reader (Dynatech).

(i) CEA concentrations are calculated from the standard curve.

Notes: 1. All dilutions employ double-distilled water.
2. All antisera should be pre-titrated by box titration, when batches are changed.

## TABLE A

Purification of CEA from spent media of HuCCl-14 Cell Line cultivated in tissue culture.

| step | % CEA* | yield (%) |
|------|--------|-----------|
| crude culture | 0.006 | 100 |
| PCA extract | 0.04 | 60-80 |
| ammonium sulfate | 0.5 | 50 |
| affinity chromatography | 100 | 35-45 |

purification factor : 16,700

1 liter spent media contained 0.2-0.8 mg. of crude CEA

*relative to total protein

0212880

Claims:

1. A process for the production of carcinoembryonic antigen (CEA), comprising the steps of

cultivating a CEA-producing cell line in a monolayer culture;

further cultivating the said cell line on a particulate microcarrier with continuous agitation of the culture medium, in at least one further cultivation step;

harvesting the medium containing the desired CEA secreted by the cells; and

purifying the CEA until iodination grade product is obtained.

2. A process according to claim 1, which is effected as a batchwise process.

3. A process according to claim 1, which is effected as a continuous process.

4. A process according to claim 1, wherein at least the further cultivating and isolating steps are effcted as part of a continuous process.

5. A process according to any one of claims 1 to 4, wherein the harvesting step is effected on only part of the spent medium, and the thus removed medium is replaced by a similar amount of fresh medium.

6. A process according to claim 5, wherein the harvesting step is repeated periodically at intervals of several days.

7. A process according to any one of the preceding claims, wherein the microcarrier is a cellulose-derived microcarrier.

8. A process according to any one of claims 1 to 7, wherein the microcarrier is rigid and is either cylindrically or spherically shaped.

9. A process according to any one of the preceding claims, wherein the monolayer cultivation step is effected for a period of 3-5 days or until confluency is reached.

10. A process according to any one of the preceding claims, wherein the culture medium for at least one step, selected from the monolayer cultivation step and the at least one further cultivation step, comprises RPMI No. 1640.

11. A process according to any one of the preceding claims, wherein said culture medium for the monolayer cultivation step comprises RPMI No. 1640 and additionally an antibiotic.

12. A process according to claim 11, wherein the antibiotic is selected from the group consisting of neomycin, penicillin, streptomycin or gentamycin.

13. A process according to any one of the preceding claims, wherein the culture medium for the monolayer cultivation step comprises RPMI No. 1640 and additionally fetal bovine serum (FBS).

14. A process according to claim 13, wherein the FBS is present in a proportion of substantially 10% of the RPMI No. 1640.

15. A process according to any one of the preceding claims, wherein the culture medium for the monolayer cultivation step comprises RPMI No. 1640 and the pH of said medium is adjusted to a value of about 7.2.

16. A process according to any one of the preceding claims, wherein at least one step, selected from the monolayer cultivation step and the at least one further cultivation step, is conducted at a temperature of 37 (+/-) 5°C.

17. A process according o claim 16, wherein said temperature is substantially 37°C.

18. A process according to any one of the preceding claims, wherein the culture medium for the monolayer cultivation step comprises RPMI No. 1640, and said step is conducted in an environment having a carbon dioxide content greater than that normally present in the atmosphere.

19. A process according to claim 18, wherein said environment comprises about 5-10% $CO_2$ in compressed air.

20. A process according to any one of the preceding claims, wherein the culture medium for the monolayer cultivation step comprises RPMI No. 1640, and following said step, the desired cells are removed from the surface culturing vessel with a trypsinization solution.

21. A process according to claim 20, wherein the trypsinization solution is a Trypsin-EDTA solution.

22. A process according to any one of the preceding claims, wherein the at least one further cultivation step is effected for a period of 3-10 days or until confluency is reached.

23. A process according to any one of the preceding claims wherein the culture medium for the at least one further cultivation step is aerated with a mixture of carbon dioxide and air.

24. A process according to any one of the preceding claims, wherein the at least one further cultivation step comprises at least two such successive steps of which the volume of operation in the second of said steps is approximately a whole number multiple of the volume of operation in the first of said steps.

25. A process according to claim 24, wherein the second volume of operation is about 5-10 times the volume of the first volume of operation.

26. A process according to any one of the preceding claims, wherein the purifying step comprises extraction with perchloric acid and rejecting the insoluble matter.

27. A process according to claim 26, wherein the extraction is followed by concentration and desalting.

28. A process according to claim 27, wherein the concentration and desalting is followed by treatment with ammonium sulfate, and rejecting the non-CEA proteins thus precipitated.

29. A process according to claim 28, wherein the treatment with ammonium sulfate is followed by concentration and desalting.

30. A process according to any one of the preceding claims, wherein the purifying step is immediately preceded by the additional steps of ultrafiltration and centrifugation.

31. A process according to any one of the preceding claims, wherein the purifying step comprises the sub-step of chromatographic purification by means of affinity chromatography.

32. A process according to claim 31, wherein said sub-step is effected by affinity chromatography on a Sepharose Monoclonal-Anti-CEA-Ab column.

33. A process according to claim 31 or 32, wherein the said sub-step is effected at about 4°C.

34. A process according to any one of the preceding claims, wherein said CEA-producing cell line is HuCCL-14A.

35. Carcinoembryonic antigen having an immunoreactivity similar to that of established CEA preparations, a molecular weight substantially in the range of about 160,000 to 200,000 and possessing the following % by weight analysis, namely: protein, 45.2 (+/- 2.3); N-acetylglucoseamine, 19.1 (+/- 0.95); mannose, 6.6 (+/- 0.33); fucose, 7.6 (+/- 0.38); galactose 9.3 (+/- 0.47); and sialic acid 12.3 (+/-0.62).

FIG 1

○ METASTATIC $^{125}$-I-CEA

● HuCCL$^{125}$ I-CEA

PERCENT BINDING (vertical axis: 20, 40, 60, 80, 100)

ANTISERUM DILUTION (horizontal axis: 1:200, 1:400, 1:800, 1:1600, 1:3200, 1:6400, 1:12800, 1:25600, 1:51200)

0212880

FIG 2

PERCENT INHIBITION

△ METASTATIC CEA

▲ HuCCL CEA

NG CEA/ML

2/2

0212880

## DOCUMENTS CONSIDERED TO BE RELEVANT

EP 86305869.9

| Category | Citation of document with indication, where appropriate, of relevant passages | Relevant to claim | CLASSIFICATION OF THE APPLICATION (Int. Cl.4) |
|---|---|---|---|
| D,A | CANCER RESEARCH, vol. 36, no. 12, December 1976 (Chicago)<br><br>A. Leibovitz et al. "Classification of Human colorectal Adenocarcinoma Cell Lines"<br>pages 4562 - 4569<br><br>    * Totality *<br><br>-- | 1 | C 12 P 21/00<br><br>C 12 N  5/00<br><br>A 61 K 39/00 |
| D,A | EXPERIMENTAL CELL BIOLOGY, vol. 46, 1978 (Basel, München, Paris, London, New York, Sydney)<br><br>A. YANIV et al. "Establishment and Characterization of Cell Line Derived from Human Colon Adenocarcinoma (HuCCL-14)"<br>pages 220-230<br><br>    * Totality *<br><br>-- | 1 | |
| A | EP - A2 - 0 119 556 (SLOAN-KETTERING INSTITUTE FOR CANCER RESEARCH)<br><br>    * Abstract *<br><br>---- | 1 | |

**TECHNICAL FIELDS SEARCHED (Int. Cl.4)**

C 12 P

C 12 N

A 61 K

The present search report has been drawn up for all claims

| Place of search | Date of completion of the search | Examiner |
|---|---|---|
| VIENNA | 07-11-1986 | WOLF |